# EUROPÄISCHE PATENTSCHRIFT

(11) **EP 1 111 112 B1**
(45) Veröffentlichungstag und Bekanntmachung des Hinweises auf die Patenterteilung: **05.03.2008**
(21) Anmeldenummer: 00125543.9
(22) Anmeldetag: 22.11.2000
(51) Int. Cl.: D04H 1/42, D04H 1/56

(54) **Medizintechnisches Produkt sowie Verfahren zu seiner Herstellung**
Medico-technical product and method of its production
Produits medico-techniques, et procédé pour sa production

(30) Priorität: 08.12.1999 DE 19959088
(43) Veröffentlichungstag der Anmeldung: 27.06.2001
(73) Patentinhaber: Deutsche Institute für Textil- und Faserforschung Denkendorf, 73770 Denkendorf (DE)
(72) Erfinder: Dauner, Martin, Dr, Ing., 73728 Esslingen (DE); Planck, Heinrich, Prof, Dr., 72622 Nürtingen (DE); Linti, Carsten, 70372 Stuttgart (DE)
(74) Vertreter: Patentanwälte Ruff, Wilhelm, Beier, Dauster & Partner

(56) Entgegenhaltungen:
- WO-A-99/17817
- US-A- 4 043 331

## Beschreibung

Die vorliegende Erfindung betrifft ein medizintechnisches Produkt sowie ein Verfahren zu seiner Herstellung.

In der medizinischen Technik sind zur Herstellung von Implantaten und Organersatz bioverträgliche Materialien erforderlich. Für spezielle Anwendungen wie etwa Gefäßprothesen oder Knorpelersatz sollen die Implantatmaterialien in eine gewünschte Form, beispielsweise einen Hohlkörper ausgebildet sein. Damit das Implantat seine Funktion im Körper optimal erfüllen kann, muß das Implantat gut einwachsen und am besten vollständig von Körperzellen besiedelt werden.

Nach herkömmlichen Techniken der Kunststoffverarbeitung hergestellte Implantate können zwar in einer gewünschten Form hergestellt werden, weisen jedoch eine nichtstrukturierte Oberfläche auf und sind daher in der Umgebung eines lebenden Körpers eher zellabweisend, so daß ein Einwachsen von Körperzellen behindert wird.

Aus Fasern oder Garnen nach textilen Techniken hergestellte Implantate in Form von Geweben, Maschenwaren oder Nonwovens weisen eine Oberflächenstruktur und Porosität auf. Hier ist wiederum die Formgebung durch die Herstellungstechnik wie Weben, Wirken, Vernadeln usw. eingeschränkt. Ferner treten bei Hohlkörpern, wie rohrförmigen Produkten, oft Probleme mit der Steifigkeit auf, so daß das Lumen kollabiert, falls der Innendruck abfällt.

Die vorliegende Erfindung stellt sich die Aufgabe, ein medizintechnisches Produkt zur Verfügung zu stellen, das aus bioverträglichem Polymermaterial hergestellt ist, mit geringem Aufwand und zu günstigen Kosten in eine beliebige Form ausgebildet werden kann und bei Verwendung in der Medizin das Zellwachstum begünstigt.

Diese Aufgabe wird gelöst durch ein medizintechnisches Produkt mit einer Melt-Blown-Faserstruktur aus bioverträglichem Polymermaterial in Form eines dreidimensionalen Formkörpers mit einer Porenstruktur, die Zellwachstum begünstigt.

Beim Melt-Blown-Verfahren wird ein thermoplastisches, zur Faserbildung geeignetes Polymer durch einen Düsenkopf gepreßt, der sehr viele, meist mehrere hundert kleine Öffnungen von üblicherweise etwa 0,4 mm Durchmesser aufweist. Um den Düsenkopf austretende und zusammenlaufende Heißgasströme von etwa 100 bis 360 °C, abhängig vom verwendeten Polymer, nehmen das faserförmige extrudierte Polymer mit, so daß es gleichzeitig verstreckt wird. Es werden dabei sehr feine Fasern von einigen Mikrometern Durchmesser erhalten. In einem starken Gasstrom werden die spinnfrischen verstreckten Fasern einer Auffangvorrichtung zugeführt, wo sich eine feine Faserschicht als luftverwirbeltes, verklebendes Vlies ausbildet. Die Haftung der Spinnfasern im Faserverbund beruht auf einer kombinierten Wirkung von Verschlingung und Verklebung der noch schmelzwarmen, nicht vollständig erstarrten Fasern.

Das erfindungsgemäße medizintechnische Produkt kann in einer Ausführungsform in Form eines Hohlkörpers vorliegen. Bevorzugt kann das medizintechnische Produkt in Form eines rohrförmigen Körpers vorliegen. Als Beispiel für einen solchen rohrförmigen Körper sind Implantate als Ersatz für Gefäße zum Transport von Körperflüssigkeiten sowie rohrförmigen Körperorganen wie Speiseröhre oder Luftröhre zu nennen.

Das erfindungsgemäße medizintechnische Produkt kann in einer anderen Ausführungsform in Form einer Freiform vorliegen. Als Beispiel für eine solche Freiform ist die Nachbildung einer Ohrmuschel als Ersatz für ein fehlendes körpereigenes Ohr zu nennen.

Mit Vorteil kann das erfindungsgemäße medizintechnische Produkt in Form mehrerer übereinanderliegender Schichten ausgebildet sein. Das heißt, ein gemäß der Erfindung ausgebildeter Formkörper weist im Querschnitt eines Materials eine Schichtstruktur aus Melt-Blown-Fasern auf. In einem solchen Schichtaufbau können unterschiedliche Polymere verwendet sein. Ferner können sich die eingesetzten Fasern in ihrem Faserdurchmesser und/oder ihren Fasereigenschaften unterscheiden. Auch können sich die einzelnen Schichten in Porosität, Porengröße und/oder Porenvolumen unterscheiden. Auf diese Weise können durch geeignete Wahl des Schichtaufbaus Funktionseigenschaften variiert werden, wie beispielsweise Degradierbarkeit oder auch Blutkompatibilität.

Ebenso kann bei einer Freiform das Material im Querschnitt eine Schichtstruktur aufweisen. Auch können flächig ausgebildete Schichten einer Melt-Blown-Faserstruktur zu einer dreidimensionalen Struktur übereinander angeordnet sein. Die Ausbildung einer Schichtstruktur ist mit Melt-Blown-Fasern besonders einfach, da durch weitere Melt-Blow-Schritte weitere Faserschichten aufgebracht werden können, die in der Schmelzwärme mit der darunterliegenden Schicht einen Verbund bilden.

Das medizintechnische Produkt mit Melt-Blown-Faserstruktur kann mit Vorteil Funktionselemente aufweisen.

Ferner kann das medizintechnische Produkt Verstärkungselemente aufweisen. Als Verstärkungselemente können beispielsweise Verstärkungsstäbe, Verstärkungsringe, Verstärkungsspangen, Verstärkungsspiralen, Verstärkungsfasern, Textilgebilde usw. allein oder in Kombination miteinander vorgesehen sein. Bevorzugt können als Werkstoffe für die Verstärkungselemente bioverträgliche Polymere, bioverträgliche Metalle, bioverträgliche Keramik und/oder bioverträgliche Verbundwerkstoffe ausgewählt sein.

Insbesondere können solche Verstärkungselemente radial eingebracht sein. Ferner können solche Verstärkungselemente axial in Umfangsrichtung verlaufend eingebracht sein. Das erfindungsgemäße medizintechnische Produkt kann sich mit besonderem Vorteil dadurch auszeichnen, daß es selbsttragend ist.

Nach einem mehrstufigen Melt-Blown-Verfahren, wie es oben für die Ausbildung von Schichtstrukturen beschrieben ist, können auch vorgesehene Verstärkungselemente einfach und zuverlässig in das medizintechnische Produkt eingebracht werden. Es werden zunächst eine oder mehrere Grundschichten aus Melt-Blown-Fasermaterial ausgebildet, dann ein oder mehrere Verstärkungselemente aufgesetzt und nochmals in einem oder mehreren Schritten nach dem Melt-Blown-Verfahren Polymerfasermaterial aufgetragen. Die Verstärkungselemente können so im medizintechnischen Produkt befestigt und in das bioverträgliche Polymermaterial eingebettet werden.

Ebenso können Membranen, z. B. Kapillarmembranen eingearbeitet sein. Ein solches medizintechnisches Produkt kann mit Vorteil als immunologische Trennmembran wirken. Gleichzeitig ist der Transport von kleinen Molekülen ermöglicht, wie es beispielsweise für den Nährstofftransport vorteilhaft ist. Ferner kann eine Membran zur Begasung, beispielsweise mit Sauerstoff oder Kohlendioxid dienen.

Zur Begünstigung des Zellwachstums auf und im medizintechnischen Produkt ist eine Porenstruktur von besonderer Bedeutung. Das erfindungsgemäße medizintechnische Produkt zeichnet sich besonders dadurch aus, daß die Porengröße der Melt-Blown-Faserstruktur mehr als 3 Mikrometer (> 3 µm) betragen kann. Insbesondere kann die Porengröße im medizintechnischen Produkt der Erfindung 10 bis 300 µm betragen. In einer besonders bevorzugten Ausführungsform kann die Porengröße im medizintechnischen Produkt der Erfindung 20 bis 100 µm betragen. Erfindungsgemäß kann die Melt-Blown-Faserstruktur eine Porosität von 50 bis 99 % aufweisen.

Das erfindungsgemäße medizintechnische Produkt kann eine für das gewählte Polymer und die gewählte Konstruktion übliche Festigkeit pro Flächengewicht aufweisen. Wenn das erfindungsgemäße medizintechnische Produkt zur Zellbesiedelung vorgesehen ist, spielt Festigkeit nur eine untergeordnete Rolle.

In einer Ausführungsform des erfindungsgemäßen medizintechnischen Produkts kann das Polymermaterial unter physiologischen Bedingungen mindestens teilweise, vorzugsweise im wesentlichen nicht resorbierbar sein.

Das Polymermaterial für das erfindungsgemäße medizintechnische Produkt kann ausgewählt sein aus der Gruppe thermoplastischer Polymere z. B. Polyurethan (PU), Polyethylenterephthalat (PET), Polybutylenterephthalat (PBT), Polyetherketone (PEK), Polysulfone (PSU), Polypropylen (PP), Polyethylen (PE), Copolymeren, Terpolymeren und/oder Mischungen davon. Es können auch elastomere Polymere eingesetzt werden.

In einer anderen Ausführungsform des erfindungsgemäßen medizintechnischen Produkts kann das Polymermaterial unter physiologischen Bedingungen mindestens teilweise resorbierbar sein. Insbesondere kann durch Wahl unterschiedlich resorbierbarer Polymere eine Variation des Degradationsverhaltens bzw. Resorptionsverhaltens erreicht werden. Ferner kann durch Wahl der Struktur des erfindungsgemäßen medizintechnischen Produkts eine Variation des Degradationsverhaltens bzw. Resorptionsverhaltens erreicht werden.

Das Polymermaterial für das erfindungsgemäße medizintechnische Produkt kann ausgewählt sein aus der Gruppe resorbierbarer thermoplastischer Polymere umfassend Polyglycolid, Polylactid, Polycaprolacton, Trimethylencarbonat, resorbierbare Polyurethane, Copolymere, Terpolymere und/oder Mischungen davon.

In einer bevorzugten Ausführungsform kann sich das medizintechnische Produkt dadurch auszeichnen, daß das Melt-Blown-Fasermaterial mindestens teilweise, vorzugsweise im wesentlichen resorbierbar ist, eingebrachte Funktionselemente und/oder Verstärkungselemente nur teilweise resorbierbar sind.

Zur Erzielung spezifischer Eigenschaften kann die Verwendung von zwei oder mehreren unterschiedlichen Polymermaterialien erforderlich sein. Hierzu können Fasern oder Partikel in den Luftstrom eingeblasen werden. In einer weiteren Variante können unterschiedliche Polymere im Extruder zu einem sogenannten Blend gemischt werden.

In einer anderen Ausführung kann ein Bi- oder Multikomponenten-Melt-Blow-Spinnkopf verwendet werden, in dem zwei oder mehrere Polymerschmelzen gleichzeitig verarbeitet werden. Hierbei können die Schmelzströme vor Verlassen der Düse (Kapillarbohrung) zusammengeführt werden. Alternativ können sie durch unterschiedliche Düsen (Kapillarbohrungen) getrennt geblasen werden, die alternierend oder in Reihe angeordnet sind. Mit Vorzug können hier Polymere unterschiedlichen Degradierverhaltens zusammen verarbeitet werden. Es können auch unterschiedliche Oberflächeneigenschaften gemeinsam erzielt werden, wie beispielsweise hydrophob und hydrophil. Auch kann eine Polymerkomponente als Binder ausgelegt sein.

Bei einer Ausführungsform eines mehrschichtigen medizintechnischen Produkts gemäß der Erfindung können im wesentlichen alle Schichten aus Melt-Blown-Fasermaterial gebildet sein.

Bei einer anderen Ausführungsform eines mehrschichtigen medizintechnischen Produkts gemäß der Erfindung kann mindestens eine Schicht eine unterschiedliche Struktur aufweisen. Beispielsweise können sich die Schichten im Grad ihrer Porosität und/oder dem Porendurchmesser unterscheiden. Auf diese Weise kann die Zugänglichkeit der Faserschichtstruktur für Zellen beeinflußt werden. So ermöglichen hochporöse Schichten von 30 bis 300 µm Porengröße ein Durchwachsen von Körpergewebe, makroporöse Schichten von 3 bis 30 µm Porengröße ein Ein-/Anwachsen von Körpergewebe, mikroporöse Schichten von < 3 µm dienen der Zellselektion und nanoporöse Schichten von < 0,2 µm Porengröße sind Bakterienfilter. Auf diese Weise kann eine Schicht für Zellen durchlässig sein, Zellen in ihrem Porenraum aufnehmen oder nur oberflächlich mit Zellen behaftet werden.

Bei der Anwendung in der Medizintechnik ist es vorteilhaft, daß das medizintechnische Produkt mit Melt-Blown-Faserstruktur für Nährstoffe und gegebenenfalls niedermolekulare Stoffwechselprodukte durchlässig ist, jedoch ein Eindringen von pathogenen Keimen auf einer, kontaminierten Bedingungen ausgesetzten, Seite nicht möglich ist.

Bei noch einer anderen Ausführungsform eines mehrschichtigen medizintechnischen Produkts gemäß der Erfindung kann mindestens eine Schicht nicht aus Melt-Blown-Fasermaterial gebildet sein. Beispielsweise kann ein nach anderen textilen Techniken hergestelltes Flächengebilde, wie ein Gewebe oder Gewirke oder eine, auch semipermeable, Filmschicht wie eine Polymer- oder Metallschicht eingebracht sein. Eine derart anders strukturierte Schicht kann beispielsweise zum Zwecke der Verstärkung und/oder als Barriere vorgesehen sein.

Mit Vorteil kann das erfindungsgemäße medizintechnische Produkt Melt-Blown-Fasermaterial mit Fasern mit einem Durchmesser von 0,1 bis 100 µm, insbesondere 5 bis 50 µm umfassen. Solche Fasern zeichnen sich durch eine Querschnittsfläche von weniger als 1 µm² bis über 200 µm² aus.

In Weiterbildung der Erfindung können in das medizintechnische Produkt medizinische Wirkstoffe eingebracht sein. Als Beispiele für solche medizinischen Wirkstoffe sind zu nennen: Medikamente, Diagnostika, antimikrobielle Wirkstoffe, Wachstumsfaktoren, Röntgenkontrastmittel, Hämostatika, Hydrogele oder Superadsorber.

Die vorliegende Erfindung stellt ferner ein Verfahren zur Verfügung zur Herstellung eines medizintechnischen Produkts nach einem Melt-Blown-Verfahren aus bioverträglichem Polymermaterial zu einem dreidimensionalen Formkörper mit einer Porenstruktur, die Zellwachstum begünstigt.

Erfindungsgemäß kann ein dreidimensionaler Körper in aufbauendem Verfahren geformt werden. Mit Vorteil kann sich das Verfahren gemäß der Erfindung dadurch auszeichnen, daß für die Herstellung des medizintechnischen Produkts eine Form, insbesondere eine Matrize verwendet wird, die mindestens teilweise durch Melt-Blown-Fasern ausgefüllt wird.

In einer anderen Ausführungsform kann sich das erfindungsgemäße Verfahren dadurch auszeichnen, daß für das medizintechnische Produkt eine grobporöse Stützstruktur, beispielsweise eine Gitterstruktur, mindestens teilweise mit Melt-Blown-Fasern ausgefüllt wird.

In noch einer weiteren Ausführungsform kann sich das erfindungsgemäße Verfahren dadurch auszeichnen, daß das medizintechnische Produkt auf eine vorgebildete Hohlform, beispielsweise eine Rohrform, mindestens teilweise mit Melt-Blown-Fasern aufgebaut wird.

Bei allen Verfahrensvarianten können nach der Melt-Blown-Technik produzierte Fasern in einer Schicht oder in mehreren Schichten aufgebracht werden. Hierbei können die einzelnen Schichten gleich dick oder unterschiedlich dick ausgebildet werden. Ebenso können Schichten in unterschiedlichen Anordnungsmustern aufgebracht werden.

Das Melt-Blown-Verfahren ist zur Herstellung der medizintechnischen Produkte gemäß der Erfindung besonders vorteilhaft, da praktisch alle Thermoplaste verarbeitbar sind. Darunter auch schwerlösliche Polymere wie Polyethylenterephthalat, Polypropylen oder Polyglykolsäure. Ferner sind keine Lösungsmittel, Additive oder andere chemische Hilfsmittel erforderlich, die sich bei Verwendung des Produkts in der Medizin als für den Patienten schädlich erweisen könnten.

Verwendung findet ein medizintechnisches Produkt aus bioverträglichem Polymermaterial gebildet aus Melt-Blown-Fasermaterial, das in Form eines dreidimensionalen Formkörpers ausgebildet ist und eine Porenstruktur aufweist, die Zellwachstum begünstigt, in der Humanmedizin und/oder Veterinärmedizin. In einer Ausführungsform kann das erfindungsgemäße medizintechnische Produkt als Implantat verwendet werden. Bevorzugt kann das Implantat als dreidimensionaler Körper die Raumform eines zu ersetzenden Körperteils aufweisen. Als besonders bevorzugtes Beispiel einer Verwendung als Implantat ist eine Tracheaprothese zum Ersatz der Luftröhre beim Patienten zu nennen.

Zur Implantation bei einem menschlichen oder tierischen Patienten vorgesehene medizintechnische Produkte können mit Vorteil in der gewünschten Form und in den für den jeweiligen Patienten angepaßten Abmessungen angefertigt werden. Bevorzugt kann das erfindungsgemäße medizintechnische Produkt zur Besiedelung mit Zellen in vitro und/oder in vivo verwendet werden. Beispielsweise kann das vorgefertigte medizintechnische Produkt in vitro mit Zellen des Patienten besiedelt werden. Danach wird das Implantat dem Patienten eingesetzt. Auf diese Weise ist ein besseres Einwachsen, schnellere Heilung und mit weniger Komplikationen zu erreichen.

In einer anderen Ausführungsform kann das erfindungsgemäße medizintechnische Produkt als extrakorporaler Organersatz verwendet werden. Als besonders bevorzugtes Beispiel einer Verwendung ist hier der Einsatz in einem Leberreaktor zum Ersatz einer funktionsuntüchtigen Leber außerhalb des Körpers des Patienten zu nennen. Für eine solche Anwendung können bevorzugt nicht resorbierbare Polymere verwendet werden.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

Die Beispiele nehmen Bezug zu den begleitenden Zeichnungen, in denen dargestellt sind:
- Figur 1: einen Längsschnitt durch eine Tracheaprothese mit erfindungsgemäßer Melt-Blown-Faserstruktur als innere und äußere Fasermaterialschicht mit eingebauten Verstärkungsspangen,

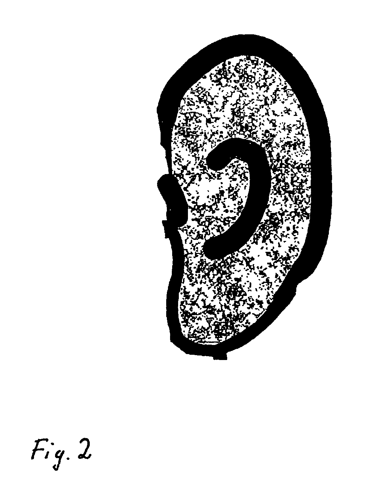

- Figur 2: eine schematische Darstellung einer menschlichen Ohrmuschel, die aus erfindungsgemäßer Melt-Blown-Faserstruktur nachgebildet ist, und
- Figur 3: eine schematische Darstellung eines Leberreaktor-Inlays mit erfindungsgemäßer Melt-Blown-Faserstruktur mit eingebauten Kapillaren für den Gasaustausch, einer großporigen Struktur zur Aufnahme von Hepatozyten und einer feinporigen Struktur für die Stoffwechselunterstützung.

### Beispiel 1

### Tracheaprothese

Für eine in einen Patienten zu implantierende Trachea wird eine rohrförmige Hohlstruktur mit hufeisenförmigen Verstärkungsspangen nach einem mehrstufigen Melt-Blown-Verfahren hergestellt.

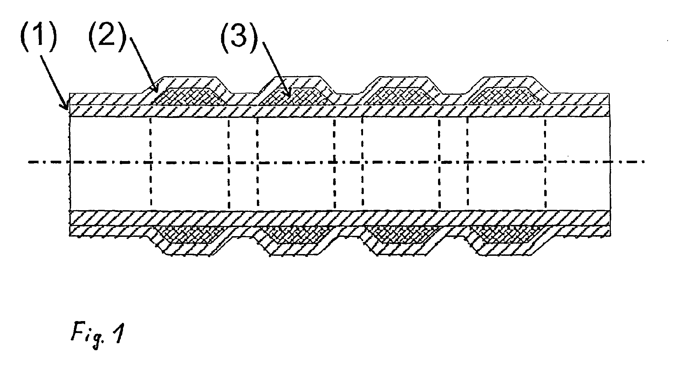

In der beigefügten Figur 1 zeigt Bezugszeichen 1 eine innere Schicht aus Melt-Blown-Fasermaterial, 2 eine äußere Schicht aus Melt-Blown-Fasermaterial und 3 eingebaute Verstärkungsspangen.

Mit Hilfe einer rohrförmigen Siebvorrichtung geeigneter Abmessungen wird zunächst eine mikroporöse Innenwandstruktur ausgebildet. Dann werden einzelne hufeisenförmige Verstärkungsspangen aus Kunststoff wie beispielsweise PUR, PET, PP aufgesetzt und mit der Innenschicht verklebt. Anschließend wird die makroporöse Außenschicht nach dem Melt-Blown-Verfahren aufgebracht.

Für die erste Schicht wird Polyurethan bei 180 °C Schmelzetemperatur mit einem Volumenstrom von 9,6 cm³/min durch die Kapillaren der Düse gepreßt. Für die Blasluft wird auf 250 °C erhitzt und bei 5,5 bar wird ein Volumenstrom von 45 Nm³/h erzeugt. Die Faserstruktur hat eine Porosität von 83 % mit Poren in der Größe von 11 µm bis 87 µm (Mittelwert 30 µm).

Für die zweite Schicht wird Polyurethan bei 180 °C aufgeschmolzen und mit einem Volumenstrom von 9,6 cm³/min durch die Kapillaren einer Düse gepreßt. Für die Blasluft wird auf 230 °C erhitzt und bei 4,75 bar wird ein Volumenstrom von 38 Nm³/h erzeugt. Die erhaltene Melt-Blown-Struktur weist eine Porosität von 84 % mit Poren in der Größe von 16 µm bis 300 µm auf (Mittelwert 82 µm).

Die Spangen sind derart, daß sie nur 270 Grad des Umfangs der Prothese verstärken und den restlichen Teil frei lassen. Dieser liegt im Patienten der Speiseröhre zugewandt und ermöglicht durch seine Nachgiebigkeit eine bessere Schluckfunktion.

Die feinporige Innenschicht ermöglicht Nährstoffaustausch mit der Umgebung, hier der Atemluft, ist jedoch undurchlässig für Bakterien, mit denen die Luft kontaminiert sein könnte. Auf der dem Körper zugewandten Seite befindet sich die großporige Außenschicht der Tracheaporthese, die ein leichtes Einwachsen von Körpergewebe begünstigt. In einer anderen Ausführungsform kann die Innenschicht zur Besiedelung mit Flimmerepithel ausgelegt sein.

Besonders wichtig ist bei einer Tracheaprothese die Formstabilität, Biegesteifigkeit und Torsionssteifigkeit. Mechanische Eigenschaften und Poreneigenschaften von nach dem Meltblown-Verfahren hergestellten Strukturen sind in der folgenden Tabelle 1 angegeben.

**Tabelle 1**

| Material | Reißspannung | Standard-abw. | Reißdehnung | Reiß-Spannung / Dichte | Porenvolumen [%] | Mittlere Poren-größe |
|---|---|---|---|---|---|---|
| | [N/mm²] | | [%] | [N*cm/g] | | [µm] |
| Polyurethan | 1,27 | 0,17 | 351 | 482,9 | 77,0 | 19,0 |
| Polyurethan | 1,01 | 0,21 | 368 | 289,9 | 69 | 30 |
| Polyurethan | 0,53 | 0,05 | 300 | 212,2 | 78 | 44 |
| Polyurethan | 0,48 | 0,15 | 267 | 202,6 | 79 | 55 |
| Polyurethan | 0,57 | 0,07 | 323 | 172,2 | 71 | 79 |
| PGA | 0,06 | 0,00 | 43 | 60,4 | 94 | 27 |
| PGA | 0,07 | 0,01 | 53 | 79,4 | 94 | 43 |
| P-L-LA | 0,24 | 0,02 | 47 | 378,0 | 95 | 28 |

### Beispiel 2

### Ohrmuschelprothese

Durch angeborene oder erworbene Defekte bis zum völligen Fehlen der Ohrmuschel entstehen schwere psychogene Störungen. Die bisherige Therapie verwendet aufwendig geschnitzte Eigentransplantate aus Rippenbögen. Die in vitro Gewebezüchtung kann hier Abhilfe schaffen. Es wird ein Gerüst benötigt, das die Form des zu züchtenden Ohres vorgibt, in der sich dann die Knorpelzellen neu organisieren können.

Eine solche Gerüstkonstruktion kann nach der Melt-Blow-Technik besonders vorteilhaft gestaltet werden, da die Fasern direkt in bzw. auf eine entsprechende Form geblasen werden können. Der auftreffende Luftstrom wird abgesaugt, wie dies beim Melt-Blow-Verfahren üblich ist. Auf die derart ausgebildete Rohform eines Ohres werden dann dem Patienten entnommene Knorpelzellen aufgebracht, die während Inkubation in vitro das Ohrgerüst aus Melt-Blown-Fasern vollständig besiedeln. Es ergibt sich so ein möglichst naturgetreues und, da körpereigene Zellen verwendet sind, besonders verträgliches Implantat. Anschließend wird die Ohrprothese dem Patienten eingesetzt.

### Beispiel 3

### Leberreaktor

Für einen als extrakorporalen zeitweiligen Leberersatz anzuwendenden Leberreaktor wird aus nicht resorbierbarem Polyurethan eine mehrschichtige Struktur aus Melt-Blown-Fasermaterial gebildet. Auf ein zunächst ausgebildetes flaches Vlies wird eine Kapillarmembran aufgelegt und dann nochmals Melt-Blown-Fasermaterial aufgespritzt.

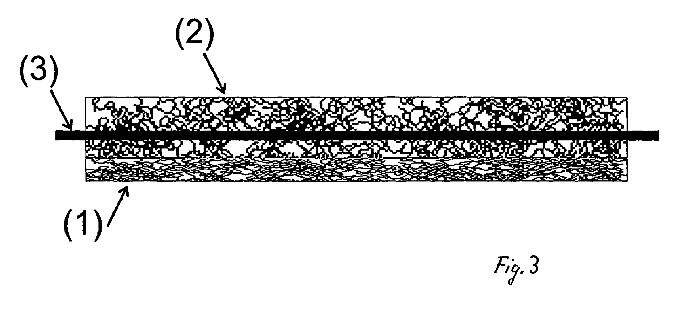

In der beigefügten Figur 3 zeigt Bezugszeichen 1 eine großporige Faserschicht, 2 eine feinporige Faserschicht und 3 eingebaute Kapillaren für den Gasaustausch.

Für die erste Schicht wird Polyurethan bei 180 °C aufgeschmolzen und mit einem Volumenstrom von 9,6 cm³/min durch die Kapillaren einer Düse gepreßt. Die Blasluft wird auf 230 °C erhitzt und bei 4,75 bar wird ein Volumenstrom von 38 Nm³/h erzeugt. Die erhaltene Melt-Blown-Struktur weist eine Porosität von 84 % mit Poren in der Größe von 16 µm bis 300 µm auf (Mittelwert 82 µm). Für die zweite Schicht wird Polyurethan bei der gleichen Schmelzetemperatur mit einem Volumenstrom von 9,6 cm³/min durch die Kapillaren der Düse gepreßt. Die Blasluft wird auf 250 °C erhitzt und bei 5,5 bar wird ein Volumenstrom von 45 Nm³/h erzeugt. Die Faserstruktur hat eine Porosität von 83 % mit Poren in der Größe von 11 µm bis 87 µm (Mittelwert 30 µm).

Der Leberreaktor umfaßt zwei Melt-Blown-Faserstrukturschichten, eine grobporige zur Aufnahme von Hepatozyten und eine feinporige zur Versorgung mit Nährstoffen, durch die Zellen nicht hindurchtreten können. Ferner sind Kapillarmembranen in der grobporigen Schicht vorgesehen, die zur Versorgung mit Sauerstoff, zum Abtransport von CO₂ oder auch als Gallengangkanäle dienen können. Die poröse Melt-Blown-Faserschichtstruktur befindet sich in einem geschlossenen Gefäß, wo sie vom zu behandelnden Plasma des Patienten durchströmt wird.

## Patentansprüche

1. Medizintechnisches Produkt mit einer Melt-Blown-Faserstruktur aus bioverträglichem Polymermaterial in Form eines dreidimensionalen Formkörpers mit einer Porenstruktur, die Zellwachstum begünstigt.

2. Medizintechnisches Produkt nach Anspruch 1, **dadurch gekennzeichnet, daß** es in Form eines Hohlkörpers vorliegt.

3. Medizintechnisches Produkt nach Anspruch 1, **dadurch gekennzeichnet, daß** es in Form einer Freiform vorliegt.

4. Medizintechnisches Produkt nach einem der vorhergehenden Ansprüche, **dadurch gekennzeichnet, daß** es in Form mehrerer übereinanderliegender Schichten ausgebildet ist.

5. Medizintechnisches Produkt nach einem der vorhergehenden Ansprüche, **dadurch gekennzeichnet, daß** es Funktionselemente aufweist.

6. Medizintechnisches Produkt nach einem der vorhergehenden Ansprüche, **dadurch gekennzeichnet, daß** es Verstärkungselemente aufweist.

7. Medizintechnisches Produkt nach einem der vorhergehenden Ansprüche, **dadurch gekennzeichnet, daß** es selbsttragend ist.

8. Medizintechnisches Produkt nach einem der vorhergehenden Ansprüche, **dadurch gekennzeichnet, daß** die Porengröße der Melt-Blown-Faserstruktur > 3 µm, bevorzugt 10 bis 300 µm beträgt.

9. Medizintechnisches Produkt nach einem der vorhergehenden Ansprüche, **dadurch gekennzeichnet, daß** die Melt-Blown-Faserstruktur eine Porosität von 50 bis 99 % aufweist.

10. Medizintechnisches Produkt nach einem der vorhergehenden Ansprüche, **dadurch gekennzeichnet, daß** das Polymermaterial unter physiologischen Bedingungen mindestens teilweise, vorzugsweise im wesentlichen nicht resorbierbar ist.

11. Medizintechnisches Produkt nach einem der Ansprüche 1 bis 11, **dadurch** gekennzeichent, daß das Polymermaterial unter physiologischen Bedingungen mindestens teilweise resorbierbar ist.

12. Medizintechnisches Produkt nach einem der vorhergehenden Ansprüche, **dadurch gekennzeichnet, daß** im mehrschichtigen Produkt im wesentlichen alle Schichten aus Melt-Blown-Fasermaterial gebildet sind.

13. Medizintechnisches Produkt nach einem der vorhergehenden Ansprüche, **dadurch gekennzeichnet, daß** im mehrschichtigen Produkt mindestens eine Schicht eine unterschiedliche Struktur aufweist.

14. Medizintechnisches Produkt nach einem der vorhergehenden Ansprüche, **dadurch gekennzeichnet, daß** im mehrschichtigen Produkt mindestens eine Schicht nicht aus Melt-Blown-Fasermaterial gebildet ist.

15. Medizintechnisches Produkt nach einem der vorhergehenden Ansprüche, **dadurch gekennzeichnet, daß** das Melt-Blown-Fasermaterial Fasern mit einem Durchmesser von 0,1 bis 100 µm, insbesondere 5 bis 50 µm umfaßt.

16. Verfahren zur Herstellung eines medizintechnischen Produkts nach einem Melt-Blow-Verfahren aus bioverträglichem Polymermaterial zu einem dreidimensionalen Formkörper mit einer Porenstruktur, die Zellwachstum begünstigt.

17. Medizintechnisches Produkt nach Anspruch 1, aus bioverträglichem Polymermaterial gebildet aus Melt-Blown-Fasermaterial, das in Form eines dreidimensionalen Formkörpers ausgebildet ist und eine Porenstruktur aufweist, die Zellwachstum begünstigt,zur Verwendung in der Humanmedizin und/oder Veterinärmedizin.

18. Medizintechnisches Produkt nach Anspruch 17 zur Verwendung als Implantat.

19. Medizintechnisches Produkt nach Anspruch 17 zur Verwendung als extrakorporaler Organersatz.

## Claims

1. Medico-technical product comprising a melt-blown fibrous structure of biocompatible polymer material in the form of a three-dimensional shaped article with a porous structure which aids cell growth.

2. Medico-technical product according to Claim 1, **characterized in that** it is in the form of a hollow article.

3. Medico-technical product according to Claim 1, **characterized in that** it is present in the form of a free form.

4. Medico-technical product according to any one of the preceding claims, **characterized in that** it is constructed in the form of a plurality of superposed layers.

5. Medico-technical product according to any one of the preceding claims, **characterized in that** it has functional elements.

6. Medico-technical product according to any one of the preceding claims, **characterized in that** it has reinforcing elements.

7. Medico-technical product according to any one of the preceding claims, **characterized in that** it is self-supporting.

8. Medico-technical product according to any one of the preceding claims, **characterized in that** the pore size of the melt-blown fibrous structure is > 3 µm and preferably 10 to 300 µm.

9. Medico-technical product according to any one of the preceding claims, **characterized in that** the melt-blown fibrous structure has a porosity of 50 to 99%.

10. Medico-technical product according to any one of the preceding claims, **characterized in that** the polymer material is at least partly and preferably essentially nonresorbable under physiological conditions.

11. Medico-technical product according to any one of Claims 1 to 11, **characterized in that** the polymer material is at least partly resorbable under physiological conditions.

12. Medico-technical product according to any one of the preceding claims, **characterized in that** essentially all the layers in the multilayered device are formed of melt-blown fibrous material.

13. Medico-technical product according to any one of the preceding claims, **characterized in that** at least one layer in the multilayered device has a different structure.

14. Medico-technical product according to any one of the preceding claims, **characterized in that** at least one layer in the multilayered device is not formed of melt-blown fibrous material.

15. Medico-technical product according to any one of the preceding claims, **characterized in that** the melt-blown fibrous material comprises fibres having a diameter of 0.1 to 100 µm and in particular 5 to 50 µm.

16. Method for the manufacture of a medico-technical product by a melt-blown process from biocompatible polymer material to give a three-dimensional shaped article with a porous structure which aids cell growth.

17. Medico-technical product according to Claim 1, of biocompatible polymer material formed from melt-blown fibrous material, constructed in the form of a three-dimensional shaped article and having a porous structure which aids cell growth, for use in human medicine and/or veterinary medicine.

18. Medico-technical product according to Claim 17 for use as an implant.

19. Medico-technical product according to Claim 17 for use as an extracorporeal organ replacement.

## Revendications

1. Produit medico-technique, ayant une structure fibreuse obtenue par fusion-soufflage, constituée d'un matériau polymère biocompatible sous forme d'un objet moulé tridimensionnel ayant une structure poreuse, qui favorise la croissance des cellules.

2. Produit medico-technique selon la revendication 1, **caractérisé en ce qu'**il se présente sous forme d'un corps creux.

3. Produit medico-technique selon la revendication 1, **caractérisé en ce qu'**il se présente sous forme d'une forme libre.

4. Produit medico-technique selon l'une des revendications précédentes, **caractérisé en ce qu'**il est réalisé sous forme de plusieurs couches superposées.

5. Produit medico-technique selon l'une des revendications précédentes, **caractérisé en ce qu'**il comprend des éléments fonctionnels.

6. Produit medico-technique selon l'une des revendications précédentes, **caractérisé en ce qu'**il comprend des éléments de renfort.

7. Produit medico-technique selon l'une des revendications précédentes, **caractérisé en ce qu'**il est autoportant.

8. Produit medico-technique selon l'une des revendications précédentes, **caractérisé en ce que** le diamètre des pores de la structure fibreuse obtenue par fusion-soufflage est > 3 µm et de préférence est de 10 à 300 µm.

9. Produit medico-technique selon l'une des revendications précédentes, **caractérisé en ce que** la structure fibreuse obtenue par fusion-soufflage présente une porosité de 50 à 99 %.

10. Produit medico-technique selon l'une des revendications précédentes, **caractérisé en ce que** le matériau polymère n'est au moins partiellement, et de préférence pour l'essentiel pas résorbable dans des conditions physiologiques.

11. Produit medico-technique selon l'une des revendications 1 à 11, **caractérisé en ce que** le matériau polymère est au moins partiellement résorbable dans des conditions physiologiques.

12. Produit medico-technique selon l'une des revendications précédentes, **caractérisé en ce que**, dans le produit multicouche, pour ainsi dire la totalité des couches sont formées d'un matériau fibreux obtenu par fusion-soufflage.

13. Produit medico-technique selon l'une des revendications précédentes, **caractérisé en ce que**, dans le produit multicouche, au moins une couche présente une structure différente.

14. Produit medico-technique selon l'une des revendications précédentes, **caractérisé en ce que**, dans le produit multicouche, au moins une couche n'est pas formée d'un matériau fibreux obtenu par fusion-soufflage.

15. Produit medico-technique selon l'une des revendications précédentes, **caractérisé en ce que** le matériau fibreux obtenu par fusion-soufflage comprend des fibres ayant un diamètre de 0,1 à 100 µm, en particulier de 5 à 50 µm.

16. Procédé de fabrication d'un produit medico-technique par un procédé de fusion-soufflage à partir d'un matériau polymère biocompatible, permettant d'obtenir un objet moulé tridimensionnel ayant une structure poreuse, qui favorise la croissance des cellules.

17. Produit medico-technique selon la revendication 1, formé à partir d'un matériau polymère biocompatible, en un matériau fibreux obtenu par fusion-soufflage, qui est réalisé sous forme d'un objet moulé tridimensionnel et présente une structure poreuse qui favorise la croissance des cellules, pour une utilisation en médecine humaine et/ou en médecine vétérinaire.

18. Produit medico-technique selon la revendication 17, pour une utilisation en tant qu'implant.

19. Produit medico-technique selon la revendication 17, pour une utilisation en tant qu'organe artificiel extracorporel.
